Europäisches Patentamt

European Patent Office

Office européen des brevets

(19)

(11) Numéro de publication : **0 064 967**
**B1**

## FASCICULE DE BREVET EUROPÉEN

(12)

(45) Date de publication du fascicule du brevet :
21.11.85

(51) Int. Cl.⁴ : **A 61 K 9/50, B 01 J 13/02**

(21) Numéro de dépôt : 82870021.1

(22) Date de dépôt : 23.04.82

(54) Procédé de préparation de particules submicroscopiques, particules ainsi obtenues et compositions pharmaceutiques les contenant.

(30) Priorité : 24.04.81 FR 8108172

(43) Date de publication de la demande :
17.11.82 Bulletin 82/46

(45) Mention de la délivrance du brevet :
21.11.85 Bulletin 85/47

(84) Etats contractants désignés :
BE CH DE FR GB IT LI LU NL SE

(56) Documents cités :
EP-A- 0 007 895
CHEMICAL ABSTRACTS, vol. 86, no. 10, 7 mars 1977
réf.: 60455n, page 348 COLUMBUS, OHIO (US)
CHEMICAL ABSTRACTS, vol. 83, no. 6, 11 août 1975
réf.: 48196j, page 305 COLUMBUS, OHIO (US)
JOURNAL OF PHARMACEUTICAL SCIENCES, vol. 68,
no. 12, décembre 1979 American Pharmaceutical
Association WASHINGTON D.C. (US) P. COUVREUR
et al.: "Adsorption of antineoplastic drugs to polyalkylcyanoacrylate nanoparticles and their release in
calf serum" pages 1521-1524
CHEMICAL ABSTRACTS, vol. 94, no. 12, 23 mars 1981
réf.: 90245b, page 409 COLUMBUS, OHIO (US)
CHEMICAL ABSTRACTS, vol. 94, no. 10, 9 mars 1981
réf.: 71390d, page 372 COLUMBUS, OHIO (US)
CHEMICAL ABSTRACTS, vol. 91, no. 22, 26 novembre
1979 réf.: 181375y, page 387 COLUMBUS, OHIO (US)
Internat. J. of Pharmaceu (1970)

(73) Titulaire : N.V. SOPAR S.A.
rue de Thyle, 3a
B-6328 Sart-Dames-Avelines (BE)

(72) Inventeur : Couvreur, Patrick
Avenue Duc Wenceslas 9
B-1300 Wavre (BE)
Inventeur : Roland, Michel
38, Rue Tomberg
B-1150 Bruxelles (BE)
Inventeur : Speiser, Peter
26, Wassbargerstrasse
CH-8127 Forch (CH)

(74) Mandataire : Plucker, Guy et al
OFFICE KIRKPATRICK 4 Square de Meeûs
B-1040 Bruxelles (BE)

## Description

La présente invention a pour objet un nouveau procédé de préparation de particules submicroscopiques formées d'un cyanoacrylate d'alkyle polymérisé et contenant une substance biologiquement active. L'invention a également pour objet lesdites particules submicroscopiques ainsi que les compositions pharmaceutiques les contenant.

Des particules submicroscopiques d'un diamètre inférieur à 500 nanomètres formées d'un polymère cyanoacrylique sont déjà connues par le brevet belge n° 869 107 qui décrit des particules submicroscopiques formées par la polymérisation micellaire d'un cyanoacrylate d'alkyle, où le terme « alkyle » désigne un radical alkyle inférieur de 1 à 4 atomes de carbone, et contenant une substance biologiquement active.

Le brevet belge n° 869 107 décrit également un procédé de préparation de ces particules submicroscopiques. Suivant ce procédé de préparation décrit, le monomère (cyanoacrylate d'alkyle) est ajouté sous agitation à une solution aqueuse d'un agent tensioactif dont le pH est ajusté à une valeur inférieure à 7, et de préférence comprise entre 2 et 3, avec un acide pharmacologiquement acceptable. Le cyanoacrylate d'alkyle polymérise alors sous forme de particules submicroscopiques ; la substance biologiquement active est introduite dans le milieu soit avant l'introduction du monomère soit après polymérisation.

Les particules submicroscopiques conformes au brevet belge n° 869 107 ont de nombreuses propriétés très précieuses en tant que vecteurs pour des substances biologiquement actives qui peuvent être, par exemple, des substances médicamenteuses à usage humain ou vétérinaire ou des produits pour le diagnostic.

Ces particules submicroscopiques connues · présentent cependant certains désavantages qui limitent leur application en médecine.

Un premier désavantage des particules submicroscopiques conformes au brevet belge n° 869 107 découle du procédé suivant lequel elles sont préparées. En effet, le procédé de préparation de ces particules submicroscopiques, tel que décrit dans ledit brevet belge, fait intervenir un agent tensio-actif.

Or, la présence d'agents tensio-actifs dans les préparations pharmaceutiques destinées à être administrées par voie parentérale n'est pas souhaitable, en raison notamment de la toxicité inhérente à ces produits.

Il est vrai que le procédé de préparation décrit dans le brevet belge n° 869 107 prévoit en particulier l'utilisation d'agents tensio-actifs non ioniques. Les agents tensio-actifs non ioniques qui peuvent ainsi être utilisés sont en général moins toxiques que les agents tensio-actifs ioniques, mais présentent néanmoins une certaine toxicité qui rend peu souhaitable leur présence dans des compositions pharmaceutiques, surtout lorsque celles-ci sont destinées à être administrées par voie parentérale. Il est à noter à ce propos que l'élimination des agents submicroscopiques, après la préparation des particules submicroscopiques, est une opération quasiimpossible ou très difficile qui nécessite le recours à des méthodes de purification extrêmement délicates (ultrafiltration, ultradialyse et/ou ultracentrifugation) peu compatibles avec une fabrication à l'échelle industrielle.

Il importe de noter en outre que dans certains cas il n'est pratiquement pas possible d'éliminer de la composition les agents tensio-actifs sans éliminer en même temps une partie sensible de la substance biologiquement active qui était adsorbée dans les particules submicroscopiques.

Un second désavantage des particules submicroscopiques conformes au brevet belge n° 869 107, qui découle également de leur procédé de préparation, réside dans le fait que ces particules sont préparées dans un milieu de réaction dont le pH est ajusté à une valeur inférieure à 7, et de préférence comprise entre 2 et 3. La présence d'un acide dans le milieu de réaction est, en général, indésirable car il devra de toute manière être neutralisé au stade final de la préparation de la composition pharmaceutique.

En outre, la présence d'un acide dans le milieu de réaction peut être nettement nuisible lorsque l'on désire fixer sur les particules submicroscopiques une substance biologiquement active qui serait altérée ou décomposée au contact d'un acide.

Il est vrai que, dans certains cas, cette difficulté pourrait être évitée en préparant les particules submicroscopiques dans un milieu acide et en n'ajoutant la substance biologiquement active qu'après avoir ajusté le pH du milieu à une valeur proche de 7. Cette manière de procéder, qui éviterait d'altérer la substance biologiquement active, n'est cependant pas sans inconvénient car la quantité de substance biologiquement active qui peut être fixée sur les particules submicroscopiques est toujours plus faible lorsque la substance biologiquement active est introduite dans le milieu après formation des particules submicroscopiques, que lorsque cette même substance est introduite dans le milieu avant l'introduction du monomère.

Outre les deux désavantages cités ci-dessus au sujet des particules submicroscopiques conformes au brevet belge n° 869 107, il faut encore noter que ces particules submicroscopiques, qui sont obtenues par la polymérisation de cyanoacrylates d'alkyle inférieurs, ne se prêtent pas à certaines applications en médecine du fait qu'elles sont trop rapidement biodégradées dans l'organisme, de telle sorte qu'elles ne conviennent pas comme vecteurs pour des substances médicamenteuses nécessitant une libération plus lente.

La présente invention remédie aux désavantages cités ci-dessus.

Conformément à la présente invention, on a découvert, de manière surprenante, que des particules submicroscopiques biodégradables d'un diamètre inférieur à 600 nanomètres (contenant au moins une substance biologiquement active) peuvent être préparées par polymérisation d'un cyanoacrylate d'alkyle dans un milieu aqueux en l'absence d'agents tensio-actifs. (La substance biologiquement active pouvant être introduite dans le milieu réactionnel avant l'introduction du monomère ou après la formation des particules submicroscopiques). Il importe de noter ici que les cyanoacrylates d'alkyle sont insolubles dans l'eau et il était dès lors tout à fait inattendu qu'un tel monomère, ajouté à un milieu aqueux non additionné d'agents tensio-actifs, puisse polymériser en formant des particules submicroscopiques d'un diamètre inférieur à 600 nanomètres.

Conformément à un mode d'exécution particulier et généralement avantageux de la présente invention, on a également découvert, de manière surprenante, que lesdites particules submicroscopiques biodégradables peuvent également être préparées par polymérisation d'un cyanoacrylate d'alkyle dans un milieu aqueux qui est non seulement exempt d'agents tensio-actifs, mais qui est également exempt d'acide. Un tel procédé est assurément nouveau et original non seulement à cause de l'absence d'agents tensio-actifs dans le milieu réactionnel, mais également à cause de l'absence d'acide. Le fait que des particules submicroscopiques d'un diamètre inférieur à 600 nanomètres puissent être préparées par addition de cyanoacrylate d'alkyle dans un milieu aqueux non acidifié est en effet surprenant, car les cyanoacrylates d'alkyle (et en tout cas les cyanoacrylates d'alkyle inférieurs) sont des monomères très réactifs et il était généralement admis qu'en l'absence d'acide ils polymérisent très rapidement dès qu'ils entrent en contact avec des substances basiques ou avec de l'eau non acidifiée.

Les procédés conformes à la présente invention permettent notamment de préparer des particules submicroscopiques au départ des cyanoacrylates d'alkyle, ou le terme « alkyle » désigne un radical alkyle linéaire ou ramifié de 5 à 12 atomes de carbone. Ces particules submicroscopiques sont nouvelles et avantageuses car, dans l'organisme humain ou animal, elles ont une vitesse de biodégradation plus faible que celle des particules submicroscopiques obtenues au départ de cyanoacrylates d'alkyle inférieur (avec un radical alkyle contenant de 1 à 4 atomes de carbone) et, de ce fait elles conviennent tout particulièrement comme vecteurs pour des substances médicamenteuses nécessitant une libération lente et progressive.

La présente invention a pour objet un procédé de préparation de particules submicroscopiques d'un diamètre inférieur à 600 nanomètres formées d'un polymère synthétique et contenant au moins une substance biologiquement active, cette préparation se faisant en l'absence d'agent tensio-actif.

Suivant une forme d'exécution de l'invention, le procédé consiste :

— à préparer une solution ou solution colloïdale aqueuse d'au moins une substance biologiquement active, cette solution ou solution colloïdale aqueuse étant exempte d'agent tensio-actif,

— à ajouter, sous agitation, à cette solution ou solution colloïdale aqueuse au moins un cyanoacrylate d'alkyle, où le terme « alkyle » désigne un radical alkyle linéaire ou ramifié de 1 à 12 atomes de carbone,

— à poursuivre l'agitation jusqu'à ce qu'en substance tout le cyanoacrylate d'alkyle introduit dans le milieu réactionnel se soit transformé en particules submicroscopiques formées de polycyanoacrylate d'alkyle.

Suivant une autre forme d'exécution de l'invention, le procédé consiste :

— à préparer une solution ou solution colloïdale aqueuse d'au moins une substance biologiquement active et d'au moins une autre substance choisie parmi les sels, les sucres, les polysaccharides et les autres substances hydrosolubles pharmaceutiquement acceptables, cette solution ou solution colloïdale aqueuse étant exempte d'agent tensio-actif et ayant une pression osmotique voisine de la pression osmotique du sérum sanguin,

— à ajouter, sous agitation, à cette solution ou solution colloïdale aqueuse au moins un cyanoacrylate d'alkyle, où le terme « alkyle » désigne un radical alkyle linéaire ou ramifié de 1 à 12 atomes de carbone,

— à poursuivre l'agitation jusqu'à ce qu'en substance tout le cyanoacrylate d'alkyle introduit dans le milieu réactionnel se soit transformé en particules submicroscopiques formées de polycyanoacrylate d'alkyle.

Suivant une autre forme d'exécution de l'invention, le procédé consiste :

— à préparer une solution ou solution colloïdale aqueuse d'une ou plusieurs substances choisies parmi les sels, les sucres, les polysaccharides et les autres substances hydrosolubles pharmaceutiquement acceptables, cette solution ou solution colloïdale aqueuse étant exempte d'agent tensio-actif et ayant une pression osmotique voisine de la pression osmotique du sérum sanguin,

— à ajouter, sous agitation, à cette solution aqueuse au moins un cyanoacrylate d'alkyle, où le terme « alkyle » désigne un radical alkyle linéaire ou ramifié de 1 à 12 atomes de carbone,

— à poursuivre l'agitation jusqu'à ce qu'en substance tout le cyanoacrylate d'alkyle introduit dans le milieu réactionnel se soit transformé en particules submicroscopiques formées de polycyanoacrylate d'alkyle,

— à ajouter à cette suspension de particules submicroscopiques une solution ou solution colloïdale aqueuse d'au moins une substance biologiquement active.

Suivant un mode d'exécution particulier de chacun des trois procédés décrits ci-dessus, la solution ou solution colloïdale aqueuse dans laquelle on ajoute le cyanoacrylate d'alkyle est exempte d'acide.

Suivant un autre mode d'exécution de chacun de ces trois procédés, ladite solution ou solution colloïdale aqueuse est ajustée à un pH inférieur à 7 avec au moins un acide pharmaceutiquement acceptable. Plus particulièrement, ce pH peut être ajusté à une valeur comprise entre 2 et 3.

Suivant une autre forme d'exécution de l'invention, le procédé consiste :

— à préparer une solution aqueuse d'au moins un acide pharmaceutiquement acceptable (plus particulièrement, le pH de cette solution peut être compris entre 2 et 3),

— à ajouter, sous agitation, à cette solution aqueuse au moins un cyanoacrylate d'alkyle, où le terme « alkyle » désigne un radical alkyle linéaire ou ramifié de 1 à 12 atomes de carbone,

— à poursuivre l'agitation jusqu'à ce qu'en substance tout le cyanoacrylate d'alkyle introduit dans le milieu réactionnel se soit transformé en particules submicroscopiques formées de polycyanoacrylate d'alkyle,

— à ajouter à cette suspension de particules submicroscopiques une solution ou solution colloïdale aqueuse d'au moins une substance biologiquement active.

Lorsque, dans l'une ou l'autre forme d'exécution de l'invention, le cyanoacrylate d'alkyle est introduit dans un milieu réactionnel acide, la suspension de particules submicroscopiques formée sera avantageusement ajustée à un pH compris entre 6 et 8, par addition d'au moins une substance basique pharmaceutiquement acceptable.

Suivant une forme d'exécution particulière de l'invention, le procédé consiste :

— à ajouter à de l'eau pure, sous agitation, au moins un cyanoacrylate d'alkyle où le terme « alkyle » désigne un radical alkyle linéaire ou ramifié de 6 à 12 atomes de carbone,

— à poursuivre l'agitation jusqu'à ce qu'en substance tout le cyanoacrylate d'alkyle introduit dans l'eau se soit transformé en particules submicroscopiques formées de polycyanoacrylate d'alkyle,

— à ajouter à cette suspension de particules submicroscopiques une solution ou solution colloïdale aqueuse d'au moins une substance biologiquement active.

Lorsque, pour la mise en œuvre de l'invention, le cyanoacrylate d'alkyle est introduit dans un milieu réactionnel acidifié, l'acide utilisé peut être, par exemple l'acide chlorhydrique, bromhydrique, iodhydrique, sulfurique, phosphorique, acétique, succinique, lactique, citrique ou tout autre acide pharmaceutiquement acceptable, pour autant que ledit acide soit compatible avec les composants du milieu et particulièrement avec la substance biologiquement active.

Le pH d'une suspension de particules submicroscopiques préparée en milieu acide peut être ajusté à une valeur comprise entre 6 et 8 par l'addition d'au moins une substance basique pharmaceutiquement acceptable, comme par exemple une solution d'hydroxyde de sodium ou une solution tampon de phosphate monopotassi-que et d'hydroxyde de sodium.

On peut ajouter au milieu réactionnel ou à la suspension de particules submicroscopiques des substances destinées à augmenter la pression osmotique, comme par exemple le chlorure de sodium, le glucose ou le dextrane. Ces substances peuvent être ajoutées après la préparation de la suspension de particules submicroscopiques, mais lorsqu'on utilise un sucre ou un polysaccharide (comme le glucose ou le dextrane) il est généralement avantageux d'introduire cette substance dans le milieu réactionnel avant que l'on y introduise le cyanoacrylate d'alkyle, car cette manière de procéder permet d'obtenir des particules submicroscopiques de diamètre moyen plus petit avec certains monomères.

Les particules submicroscopiques suivant l'invention peuvent contenir une ou plusieurs substances biologiquement actives. Les substances biologiquement actives que peuvent contenir les particules submicroscopiques sont, par exemple, les substances médicamenteuses à usage humain ou vétérinaire ou les produits pour le diagnostic. Comme substances médicamenteuses, on peut citer plus particulièrement les produits chimiques doués de propriétés pharmacologiques et, par exemple, les substances antimitotiques ou antinéoplasiques comme le méthotrexate, l'actinomycine D, la doxorubicine, la daunorubicine, la bléomycine et la vincristine, ou les substances antibiotiques, comme les pénicillines, les céphalosporines et l'acide nalidixique, les antibiotiques du type aminoglucoside et ceux de la famille de la virginiamycine, ou les substances hormonales, notamment les hormones stéroïdiennes. Ces substances médicamenteuses peuvent être notamment des composés chimiques à haut poids moléculaire comme l'insuline et l'héparine, et l'expression « substance médicamenteuse » comprend également des produits biologiques comme les antigènes, les allergènes, les enzymes, les protéines, les virus, des constituants de virus, des constituants de bactéries ou des constituants de cellules. Les particules submicroscopiques suivant l'invention peuvent également contenir un produit pour le diagnostic comme par exemple la fluorescéine ou la séralbumine humaine radio-active.

L'invention a également pour objet les compositions pharmaceutiques qui contiennent des particules submicroscopiques contenant au moins une substance biologiquement active, obtenues suivant l'un ou l'autre des procédés conformes à l'invention.

Les compositions pharmaceutiques suivant l'invention peuvent contenir un excipient pour l'administration orale ou parentérale.

L'invention a également pour objet les compositions pharmaceutiques qui consistent en une suspension aqueuse de particules submicroscopiques telle qu'elle est obtenue par l'un ou l'autre des procédés de l'invention.

Suivant une réalisation avantageuse de l'invention, les compositions pharmaceutiques contiennent des particules submicroscopiques dont le diamètre moyen est inférieur à 200 nanomètres et de préférence inférieur à 100 nanomètres.

De telles compositions pharmaceutiques conviennent tout spécialement pour l'administration par voie parentérale et plus particulièrement par voie intraveineuse.

Les exemples suivants illustrent la présente invention sans aucunement en limiter la portée.

On remarquera que les exemples 1 à 8 concernent en quelque sorte des essais « à blanc », puisqu'ils décrivent la préparation de particules submicroscopiques exemptes de substance biologiquement active. On comprendra toutefois que des particules submicroscopiques contenant une substance biologiquement active peuvent être préparées de manière analogue en introduisant dans le milieu de réaction ladite substance biologiquement active, soit avant l'introduction du cyanoacrylate, soit après la formation des particules submicroscopiques et cela d'une manière analogue à ce qui est décrit aux exemples 9 à 13.

Dans tous les exemples cités ci-dessous, la préparation des particules submicroscopiques se fait à la température ambiante (environ 20 °C).

Il est à noter d'autre part que pour tous les exemples cités ci-dessous, la taille des particules submicroscopiques a été mesurée avec un compteur à diffraction d'un rayon laser (Nanosizer®). Il importe de savoir que les résultats des mesures obtenus ainsi doivent être considérés comme surestimés par rapport aux résultats qui seraient obtenus par une granulométrie au microscope électronique à balayage.

Exemple 1

Dans 25 ml d'eau distillée on ajoute, sous agitation, 50 microlitres de cyanoacrylate d'hexyle. Après 4 jours de polymérisation, on ajoute à nouveau 50 microlitres de cyanoacrylate d'hexyle. Après un nouveau délai de 4 jours, 50 microlitres de cyanoacrylate d'hexyle sont à nouveau ajoutés et on laisse la polymérisation se poursuivre pendant une nouvelle période de 4 jours. Il est à noter que l'agitation a été maintenue depuis le début de la préparation. La suspension de particules submicroscopiques ainsi obtenue présente en effet Tyndall caractéristique des solutions colloïdales. Cette suspension est filtrée sur filtre en verre fritté (diamètre des pores : 9 à 15 micromètres). Mesurées à l'aide d'un compteur à diffraction d'un rayon laser (Nanosizer®), les particules contenues dans le filtrat présentent un diamètre moyen de 80 nanomètres.

Exemple 2

On procède suivant la technique décrite dans l'exemple 1, mais l'eau distillée est remplacée par une solution de glucose à 5 % afin de rendre la suspension isotonique et donc propre à être injectée telle quelle.

Le diamètre moyen des nanoparticules ainsi obtenues est de 170 nanomètres.

Exemple 3

100 mg de dextrane 70 et 200 mg d'acide citrique sont dissous successivement dans 10 ml d'eau distillée. 120 microlitres de cyanoacrylate d'hexyle sont ensuite ajoutés, goutte à goutte. Après filtration, on obtient des particules dont le diamètre moyen est de l'ordre de 300 nanomètres.

Exemple 4

100 mg d'acide citrique et 100 mg de dextrane sont dissous dans 10 ml d'eau distillée. 120 microlitres de cyanoacrylate de méthyle sont ajoutés goutte à goutte. Après 2 heures de polymérisation, on filtre sur un filtre en verre fritté (diamètre des pores : 9 à 15 micromètres). Les particules ainsi obtenues ont un diamètre moyen d'environ 600 nanomètres.

Exemple 5

On procède suivant la technique décrite dans l'exemple 4, mais le cyanoacrylate de méthyle est remplacé par le cyanoacrylate d'isobutyle. Les particules ainsi obtenues ont un diamètre moyen d'environ 450 nanomètres.

Exemple 6

500 mg de glucose sont dissous dans 10 ml d'eau distillée. 120 microlitres de cyanoacrylate de méthyle sont ajoutés goutte à goutte. Après 2 heures de polymérisation, on filtre sur un filtre en verre fritté (diamètre des pores : 9 à 15 micromètres). Les particules ainsi obtenues ont un diamètre moyen de 160 nanomètres et la suspension est isotonique et prête à être injectée.

Exemple 7

On procède suivant la technique décrite dans l'exemple 6, mais le cyanoacrylate de méthyle est remplacé par le cyanoacrylate d'éthyle. Les particules ainsi obtenues ont un diamètre moyen de 150 nanomètres.

Exemple 8

On procède suivant la technique décrite dans l'exemple 6, mais le cyanoacrylate d'isobutyle. Les particules ainsi obtenues ont un diamètre moyen de 180 nanomètres.

Exemple 9

100 mg d'acide citrique et 100 mg de dextrane 70 sont dissous dans 10 ml d'eau distillée. 100 microlitres de cyanoacrylate d'hexyle sont ajoutés goutte à goutte, sous agitation, et cette agitation est maintenue pendant 4 jours. Après neutralisation par NaOH N, jusqu'à pH 7, on ajoute à la suspension ainsi obtenue 1 mg d'actinomycine D et 5 microlitres de solution d'actino- mycine D tritiée (activité spécifique : 14 Ci/millimole ; concentration radio-active : 0,5 mCi/ml). L'agitation est continuée pendant 2 heures et la suspension ainsi obtenue est ensuite centrifugée à 50 000 g. Par le dosage de la radio-activité (scintillateur liquide) dans le liquide surnageant et dans le culot de centrifugation, on constate que la quantité d'actinomycine D fixée sur les particules submicroscopiques correspond à 50,2 % de la quantité totale engagée.

Le diamètre moyen des particules obtenues est de 260 nanomètres.

Exemple 10

200 mg d'acide citrique sont dissous dans 10 ml d'eau distillée. 120 microlitres de cyanoacrylate d'hexyle sont ajoutés, goutte à goutte, sous agitation, et cette agitation est maintenue pendant 4 jours. Après neutralisation par NaOH N, jusqu'à pH 7, on ajoute à la suspension ainsi obtenue 5 mg de vincristine et 50 microlitres de solution de vincristine tritiée (activité spécifique : 4,5 Ci/millimole ; concentration radio-active : 0,25 mCi/ml). L'agitation est continuée pendant 2 heures et la suspension ainsi obtenue est ensuite centrifugée à 50 000 g. Par le dosage de la radio-activité (scintillateur liquide) dans le liquide surnageant et dans le culot de centrifugation, on constate que la quantité de vincristine fixée sur les particules submicroscopiques correspond à 69 % de la quantité totale engagée.

Le diamètre moyen des particules obtenues est de 150 nanomètres.

Exemple 11

200 mg d'acide citrique sont dissous dans 10 ml d'eau distillée. 120 microlitres de cyanoacrylate d'hexyle sont ajoutés, goutte à goutte, sous agitation, et cette agitation est maintenue pendant 4 jours. Après neutralisation par NaOH N, jusqu'à pH 7, on ajoute à la suspension ainsi obtenue 100 U. I. d'insuline porcine et 10 microlitres d'insuline marquée à l'iode 125 (activité spécifique : 50 μCi/mg ; concentration radio-active 1 μCi/ml). L'agitation est continuée pendant 2 heures et la suspension ainsi obtenue est ensuite centrifugée à 50 000 g. Par le dosage de la radio-activité (scintillateur gamma) dans le liquide surnageant et dans le culot de centrifugation, on constate que la quantité d'insuline porcine fixée sur les particules submicroscopiques correspond à 93 % de la quantité totale engagée.

Le diamètre moyen des particules obtenues est de 150 nanomètres.

Exemple 12

500 mg de glucose sont dissous dans 10 ml d'eau distillée. Deux fois 40 microlitres de cyanoacrylate d'hexyle sont ajoutés, sous agitation, à 4 jours d'intervalle. L'agitation est maintenue jusqu'au quatrième jour après la seconde addition de monomère. 10 mg de doxorubicine sont ensuite ajoutés. Dans ces conditions, et après filtration, on obtient des particules d'un diamètre moyen de 190 nanomètres fixant une quantité de doxorubicine de l'ordre de 60 % de la quantité totale mise en œuvre.

Exemple 13

100 mg de dextrane 70 et 50 mg d'acide citrique sont dissous successivement dans 10 ml d'eau distillée. 10 mg de $CaCl_2$ sont ensuite dissous dans le même milieu afin d'éviter une éventuelle hypocalcémie lorsque la composition pharmaceutique sera injectée. On dissout alors dans le même milieu 10 mg de doxorubicine. 100 microlitres de cyanoacrylate d'isobutyle sont enfin ajoutés, goutte à goutte, sous agitation, au milieu réactionnel. Après 4 heures de polymérisation sous agitation, on tamponne la préparation à pH 7 par NaOH N et on isotonise la suspension par 72 mg de NaCl. La préparation est prête à être administrée par voie intraveineuse et les particules ainsi obtenues ont un diamètre moyen de 140 nanomètres.

Après centrifugation à 50 000 g de la suspension ultrafine et dosage fluorimétrique de la doxorubicine dans le liquide surnageant et dans le culot de centrifugation, on constate que la quantité de doxorubicine fixée sur les particules correspond à 94 % de la quantité totale engagée.

Plusieurs mesures de toxicité sont effectuées avec des suspensions de particules obtenues suivant ce procédé. Quel que soit le schéma d'administration la toxicité inhérente à la doxorubicine est sensiblement diminuée lorsque celle-ci est adsorbée par les particules submicroscopiques.

Ainsi, par exemple, après administration intraveineuse de trois doses successives de 10 mg/kg/j de doxorubicine libre, 27,5 % des souris ne survivent pas à 15 jours. Par contre, lorsqu'on administre à des souris la même quantité de doxorubicine fixée sur des particules submicroscopiques, aucune mortalité n'est enregistrée après 15 jours.

De même, aucune souris ne survit à trois injections de 12,5 mg/kg/j de doxorubicine libre alors que lorsque ce médicament est fixé sur des particules submicroscopiques et administré aux mêmes doses, 30 % des souris survivent.

Parallèlement, la perte de poids des souris traitées par la doxorubicine fixée sur des particules submicroscopiques est sensiblement plus faible que la perte de poids des souris traitées par la doxorubicine libre.

La toxicité inhérente à la doxorubicine limite fortement les doses qui peuvent être administrées et donc l'efficacité thérapeutique. Les essais décrits ci-dessus montrent que la fixation de ce médicament cytostatique sur des particules submicroscopiques conformes à l'invention en diminue la toxicité, ce qui permet ainsi d'augmenter les doses et de ce fait l'effet thérapeutique du médicament.

## Revendications

1. Procédé de préparation de particules submicroscopiques d'un diamètre inférieur à 600 nanomètres formées d'un polymère synthétique et contenant au moins une substance biologiquement active, caractérisé en ce qu'il consiste à ajouter, sous agitation, à un milieu de réaction aqueux exempt d'agent tensio-actif, au moins un cyanoacrylate d'alkyle, où le terme « alkyle » désigne un radical alkyle linéaire ou ramifié de 1 à 12 atomes de carbone, l'agitation étant poursuivie jusqu'à ce qu'en substance tout le cyanoacrylate d'alkyle, introduit dans le milieu réactionnel se soit transformé en particules submicroscopiques formées de polycyanoacrylate d'alkyle, au moins une substance biologiquement active, choisie parmi les substances médicamenteuses à usage humain ou vétérinaire et les produits pour le diagnostic, étant introduite dans le milieu réactionnel avant l'introduction du monomère ou après la formation des particules submicroscopiques.

2. Procédé suivant la revendication 1, caractérisé en ce que le milieu de réaction aqueux dans lequel on ajoute au moins un cyanoacrylate d'alkyle, est une solution ou solution colloïdale aqueuse d'au moins une substance biologiquement active choisie parmi les substances médicamenteuses à usage humain ou vétérinaire et les produits pour le diagnostic.

3. Procédé suivant la revendication 1, caractérisé en ce que le milieu de réaction aqueux dans lequel on ajoute au moins un cyanoacrylate d'alkyle, est une solution ou solution colloïdale aqueuse d'au moins une substance biologiquement active, choisie parmi les substances médicamenteuses à usage humain ou vétérinaire et les produits pour le diagnostic, et d'au moins une autre substance choisie parmi les sels, les sucres, les polysaccharides et les autres substances hydrosolubles pharmaceutiquement acceptables, cette solution ou solution colloïdale aqueuse ayant une pression osmotique voisine de la pression osmotique du sérum sanguin.

4. Procédé suivant la revendication 1, caractérisé en ce que le milieu de réaction aqueux dans lequel on ajoute au moins un cyanoacrylate d'alkyle, est une solution ou solution colloïdale aqueuse d'au moins une substance choisie parmi les sels, les sucres, les polysaccharides et les autres substances hydrosolubles pharmaceutiquement acceptables, cette solution ou solution colloïdale aqueuse ayant une pression osmotique voisine de la pression osmotique du sérum sanguin, au moins une substance biologiquement active, choisie parmi les substances médicamenteuses à usage humain ou vétérinaire et les produits pour le diagnostic étant introduits dans le milieu réactionnel après la formation des particules submicroscopiques.

5. Procédé suivant l'une quelconque des revendications 1 à 4, caractérisé en ce que le milieu de réaction aqueux dans lequel on ajoute au moins un cyanoacrylate d'alkyle, est exempt d'acide.

6. Procédé suivant l'une quelconque des revendications 1 à 4, caractérisé en ce que, avant qu'on y ajoute au moins un cyanoacrylate d'alkyle, le milieu de réaction aqueux est ajusté à un pH inférieur à 7 avec au moins un acide pharmaceutiquement acceptable.

7. Procédé suivant la revendication 6, caractérisé en ce que, avant qu'on y ajoute au moins un cyanoacrylate d'alkyle, le milieu de réaction aqueux est ajusté à un pH compris entre 2 et 3 avec au moins un acide pharmaceutiquement acceptable.

8. Procédé suivant la revendication 1, caractérisé en ce que le milieu de réaction aqueux dans lequel on ajoute au moins un cyanoacrylate d'alkyle, est une solution aqueuse d'au moins un acide pharmaceutiquement acceptable, au moins une substance biologiquement active, choisie parmi les substances médicamenteuses à usage humain ou vétérinaire et les produits pour le diagnostic, étant introduite dans le milieu réactionnel après la formation des particules submicroscopiques.

9. Procédé suivant la revendication 8, caractérisé en ce que ladite solution aqueuse d'au moins un acide pharmaceutiquement acceptable a un pH compris entre 2 et 3.

10. Procédé suivant l'une quelconque des revendications 6 à 9, caractérisé en ce que, après la formation de la suspension de particules submicroscopiques, le pH de cette suspension est ajusté à une valeur comprise entre 6 et 8, par addition d'au moins une substance basique pharmaceutiquement acceptable.

11. Procédé suivant la revendication 1, caractérisé en ce que le milieu de réaction aqueux dans lequel on ajoute au moins un cyanoacrylate d'alkyle, est de l'eau pure, et le terme « alkyle » désigne un radical d'alkyle linéaire ou ramifié de 6 à 12 atomes de carbone, au moins une substance biologiquement active, choisie parmi les substances médicamenteuses à usage humain ou vétérinaire et les produits pour le diagnostic étant introduite dans le milieu réactionnel après la formation des particules submicroscopiques.

12. Composition pharmaceutique, caractérisée en ce qu'elle contient des particules submicroscopiques contenant au moins une substance biologiquement active choisie parmi les substances médicamenteuses à usage humain ou vétérinaire et les produits pour le diagnostic, obtenues par un procédé suivant l'une quelconque des revendications 1 à 11.

13. Composition pharmaceutique suivant la revendication 12, caractérisée en ce qu'elle contient un excipient pour l'administration orale ou parentérale.

14. Composition pharmaceutique, caractérisée en ce qu'elle consiste en une suspension aqueuse de particules submicroscopiques telle qu'obtenue par un procédé suivant l'une quelconque des revendications 1 à 11.

15. Composition pharmaceutique suivant l'une quelconque des revendications 12 à 14, caractérisée en ce que lesdites particules ont un diamètre moyen inférieur à 200 nanomètres.

16. Composition pharmaceutique suivant la revendication 15, caractérisée en ce que lesdites particules ont un diamètre moyen inférieur à 100 nanomètres.

**Claims**

1. A process for the preparation of submicroscopic particles having a diameter of less than 600 nanometers, made of a synthetic polymer and containing at least one biologically active substance, which comprises preparing an aqueous reaction medium free of surface-active agent, adding under stirring at least one alkyl cyanoacrylate, wherein the term « alkyl » denotes a linear or branched alkyl radical having 1 to 12 carbon atoms, stirring being continued until substantially all the alkyl cyanoacrylate introduced into the reaction medium has been converted into submicroscopic particles made of polyalkyl cyanoacrylate, at least one biologically active substance, selected from the medical substances for human or veterinary use and diagnostic products, being introduced into the reaction medium before the introduction of monomer or after the formation of submicroscopic particles.

2. A process as claimed in claim 1, wherein the aqueous reaction medium, to which at least one alkyl cyanoacrylate is added, is an aqueous solution or aqueous colloidal solution containing at least one biologically active substance selected from the medical substances for human or veterinary use and diagnostic products.

3. A process as claimed in claim 1, wherein the aqueous reaction medium, to which at least one alkyl cyanoacrylate is added, is an aqueous solution or an aqueous colloïdal solution containing at least one biologically active substance selected from the medical substances for human or veterinary use and diagnostic products and at least one other substance selected from salts, sugars, polysaccharides and other pharmaceutically acceptable watersoluble substances, this aqueous solution or aqueous colloïdal solution having an osmotic pressure similar to the blood serum osmotic pressure.

4. A process as claimed in claim 1, wherein the aqueous reaction medium to which at least one alkyl cyanoacrylate is added, is an aqueous solution or an aqueous colloïdal solution containing at least one substance selected from salts, sugars, polysaccharides and other pharmaceutically acceptable water-soluble substances, this aqueous solution or aqueous colloïdal solution having an osmotic pressure similar to the blood serum osmotic pressure ; at least one biologically active substance selected from the medical substances for human or veterinary use and diagnostic products being introduced in the reaction medium after the formation of submicroscopic particles.

5. A process as claimed in any of claims 1 to 4, wherein the aqueous reaction medium to which at least one alkyl cyanoacrylate is added is free of acid.

6. A process as claimed in any of claims 1 to 4, wherein, before at least one alkyl cyanoacrylate is added thereto, the aqueous reaction medium is adjusted to a pH lower than 7 with at least one pharmaceutically acceptable acid.

7. A process as claimed in claim 6, wherein before the alkyl cyanoacrylate is added thereto, the aqueous reaction medium is adjusted to a pH between 2 and 3 with at least one pharmaceutically acceptable acid.

8. A process as claimed in claim 1, wherein the aqueous reaction medium, to which at least one alkyl cyanoacrylate is added, is an aqueous solution, at least one pharmaceutically acceptable acid, at least one biologically active substance selected from the medical substances for human or veterinary use and diagnostic products being introduced into the reaction medium after the formation of submicroscopic particles.

9. A process as claimed in claim 8, wherein the said aqueous solution of at least one pharmaceutically acceptable acid has a pH between 2 and 3.

10. A process as claimed in any of claims 6 to 9, wherein, after the formation of the suspension of submicroscopic particles, the pH of this suspension is adjusted to a value of between 6 and 8 by adding at least one pharmaceutically acceptable alkaline substance.

11. A process as claimed in claim 1, wherein the aqueous reaction medium, to which at least one alkyl cyanoacrylate is added, is pure water and the term « alkyl » denotes a linear or branched alkyl radical having 6 to 12 carbon atoms, at least one biologically active substance selected from the medical substances for human or veterinary use and diagnostic products being introduced in the reaction medium after the formation of submicroscopic particles.

12. A pharmaceutical composition, which contains submicroscopic particles containing at least one biologically active substance selected from the medical substances for human of veterinary use and diagnostic products, obtained by a process as claimed in any of claims 1 to 11.

13. A pharmaceutical composition as claimed in claim 12, which contains a carrier for oral or parenteral administration.

14. A pharmaceutical composition, which consists of an aqueous suspension of submicroscopic particles, obtained by a process as claimed in any of claims 1 to 11.

15. A pharmaceutical composition as claimed in any of claims 12 to 14, wherein the said particles have an average diameter less than 200 nanometers.

16. A pharmaceutical composition as claimed in claim 15, wherein the said particles have an average diameter less than 100 nanometers.

**Patentansprüche**

1. Verfahren zur Herstellung von submikroskopischen Teilchen mit einem Durchmesser unterhalb 600 Nanometer, die aus einem synthetischen Polymeren gebildet sind und wenigstens eine biologisch aktive Substanz enthalten, dadurch gekennzeichnet, daß es darin besteht, unter Rühren zu einem wäßrigen Reaktionsmilieu, das frei von oberflächenaktivem Mittel ist, wenigstens einen Cyanoacrylsäurealkylester zuzusetzen, wobei der Ausdruck « Alkyl » einen linearen oder verzweigten Alkylrest mit 1 bis 12 Kohlenstoffatomen bedeutet, wobei das Rühren fortgesetzt wird, bis im wesentlichen der gesamte Cyanoacrylsäurealkylester, der in das Reaktionsmilieu eingeführt wurde, in submikroskopische Teilchen, gebildet aus Polyalkylcyanoacrylat, überführt ist, wobei wenigstens eine biologisch aktive Substanz, ausgewählt unter den Arzneimittelsubstanzen zur Human- oder Veterinärverwendung und den Produkten für die Diagnose, in das Reaktionsmilieu vor der Einführung des Monomeren oder nach der Bildung der submikroskopischen Teilchen eingeführt wird.

2. Verfahren gemäß Anspruch 1, dadurch gekennzeichnet, daß das wäßrige Reaktionsmilieu, in das man wenigstens einen Cyanoacrylsäurealkylester zufügt, eine wäßrige Lösung oder wäßrige kolloidale Lösung von wenigstens einer biologisch aktiven Substanz, ausgewählt unter den Arzneimittelsubstanzen zur Human- oder Veterinärverwendung und den Produkten für die Diagnose, ist.

3. Verfahren gemäß Anspruch 1, dadurch gekennzeichnet, daß das wäßrige Reaktionsmilieu, in das wenigstens ein Cyanoacrylsäurealkylester zugesetzt wird, eine wäßrige Lösung oder wäßrige kolloidale Lösung wenigstens einer biologisch aktiven Substanz, ausgewählt unter den Arzneimittelsubstanzen zur Human oder Veterinärverwendung und den Produkten für die Diagnose, und wenigstens einer anderen Substanz, ausgewählt unter den pharmazeutisch annehmbaren, wasserlöslichen Salzen, Zuckern, Polysacchariden und anderen wasserlöslichen Substanzen, ist, wobei diese wäßrige Lösung oder wäßrige kolloidale Lösung einen osmotischen Druck hat, der nahe dem osmotischen Druck des Blutserums liegt.

4. Verfahren gemäß Anspruch 1, dadurch gekennzeichnet, daß das wäßrige Reaktionsmilieu, in das man wenigstens einen Cyanoacrylsäurealkylester gibt, eine wäßrige Lösung oder wäßrige kolloidale Lösung von wenigstens einer Substanz, ausgewählt unter den pharmazeutisch annehmbaren wasserlöslichen Salzen, Zuckern, Polysacchariden und anderen Substanzen, ist, wobei diese wäßrige Lösung oder wäßrige kolloidale Lösung einen osmotischen Druck nahe bei dem osmotischen Druck des Blutserums hat, wobei wenigstens eine biologisch aktive Substanz, ausgewählt unter den Arzneimittelsubstanzen zur Human- oder Veterinärverwendung und den Produkten für die Diagnose, in das Reaktionsmilieu nach der Bildung der submikroskopischen Teilchen eingeführt wird.

5. Verfahren gemäß einem der Ansprüche 1 bis 4, dadurch gekennzeichnet, daß das wäßrige Reaktionsmilieu, in das man wenigstens einen Cyanoacrylsäurealkylester zugibt, frei von Säure ist.

6. Verfahren gemäß einem der Ansprüche 1 bis 4, dadurch gekennzeichnet, daß bevor man wenigstens einen Cyanoacrylsäurealkylester zugibt, das wäßrige Reaktionsmilieu auf ein pH unterhalb 7 mit wenigstens einer pharmazeutisch annehmbaren Säure eingestellt wird.

7. Verfahren gemäß Anspruch 6, dadurch gekennzeichnet, daß, bevor man wenigstens einen Cyanoacrylsäurealkylester zugibt, das wäßrige Reaktionsmilieu auf ein pH zwischen 2 und 3 mit wenigstens einer pharmazeutisch annehmbaren Säure eingestellt wird.

8. Verfahren gemäß Anspruch 1, dadurch gekennzeichnet, daß das wäßrige Reaktionsmilieu, in das man wenigstens einen Cyanoacrylsäurealkylester zusetzt, eine wäßrige Lösung wenigstens einer pharmazeutisch annehmbaren Säure ist, wobei wenigstens eine biologisch aktive Substanz, ausgewählt unter den Arzneimittelsubstanzen zur Human- oder Veterinärverwendung und den Produkten für die Diagnose, in das Reaktionsmilieu nach der Bildung der submikroskopischen Teilchen eingeführt wird.

9. Verfahren gemäß Anspruch 8, dadurch gekennzeichnet, daß die genannte wäßrige Lösung wenigstens einer pharmazeutisch annehmbaren Säure ein pH zwischen 2 und 3 hat.

10. Verfahren gemäß einem der Ansprüche 6 bis 9, dadurch gekennzeichnet, daß nach der Bildung der Suspension der submikroskopischen Teilchen das pH dieser Suspension auf einen Wert zwischen 6 und 8 durch Zugabe von wenigstens einer pharmazeutisch annehmbaren basischen Substanz eingestellt wird.

11. Verfahren gemäß Anspruch 1, dadurch gekennzeichnet, daß das wäßrige Reaktionsmilieu, in das man wenigstens einen Cyanoacrylsäurealkylester gibt, reines Wasser ist und der Ausdruck « Alkyl » einen linearen oder verzweigten Alkylrest mit 6 bis 12 Kohlenstoffatomen bedeutet, wobei wenigstens eine biologisch aktive Substanz, ausgewählt unter den Arzneimittelsubstanzen zur Human- oder Veterinärverwendung und den Produkten für die Diagnose, in das Reaktionsmilieu nach der Bildung der submikroskopischen Teilchen eingeführt wird.

12. Pharmazeutische Zusammensetzung, dadurch gekennzeichnet, daß sie submikroskopische Teilchen enthält, die wenigstens eine biologisch aktive Substanz, ausgewählt unter den Arzneimittelsubstanzen zur Human- oder Veterinärverwendung und den Produkten für die Diagnose, enthalten, erhalten nach einem Verfahren gemäß einem der Ansprüche 1 bis 11.

13. Pharmazeutische Zusammensetzung gemäß Anspruch 12, dadurch gekennzeichnet, daß sie einen Exzipienten zur oralen oder parenteralen Verabreichung enthält.

14. Pharmazeutische Zusammensetzung, dadurch gekennzeichnet, daß sie aus einer wäßrigen Suspension submikroskopischer Teilchen besteht, wie sie nach einem Verfahren gemäß einem der Ansprüche 1 bis 11 erhalten wurde.

15. Pharmazeutische Zusammensetzung gemäß einem der Ansprüche 12 bis 14, dadurch gekennzeichnet, daß diese Teilchen einen mittleren Durchmesser unterhalb 200 Nanometer haben.

ben.

16. Pharmazeutische Zusammensetzung gemäß Anspruch 15, dadurch gekennzeichnet, daß diese Teilchen einen mittleren Durchmesser unterhalb 100 Nanometer haben.